# EUROPEAN PATENT SPECIFICATION

(11) **EP 3 143 530 B1**
(45) Date of publication and mention of the grant of the patent: **27.03.2019**
(21) Application number: 14766671.3
(22) Date of filing: 05.09.2014
(51) Int. Cl.: G16H 40/63, G16H 50/70, G16H 50/20

(54) **INFERENCE TRANSPARENCY SYSTEM FOR IMAGE-BASED CLINICAL DECISION SUPPORT SYSTEMS**
INFERENZTRANSPARENZSYSTEM FÜR SYSTEME ZUR UNTERSTÜTZUNG BILDBASIERTER KLINISCHER ENTSCHEIDUNGEN
SYSTÈME DE TRANSPARENCE D'INFÉRENCE POUR SYSTÈMES D'AIDE À LA DÉCISION CLINIQUE À BASE D'IMAGES

(30) Priority: 16.05.2014 WO PCT/EP2014/001330
(43) Date of publication of application: 22.03.2017
(73) Proprietor: Brainlab AG, 81829 München (DE)
(72) Inventor: VÁRKUTI, Bálint, 81541 Munich (DE)
(74) Representative: SSM Sandmair
(86) International application number: PCT/EP2014/068938
(87) International publication number: WO 2015/172853

(56) References cited:
- EP-A2- 2 575 067
- WO-A1-2011/068475
- US-A1- 2010 125 462
- MEGALOOIKONOMOU V ET AL: "Medical Data Fusion for Telemedicine", IEEE ENGINEERING IN MEDICINE AND BIOLOGY MAGAZINE, IEEE SERVICE CENTER, PISACATAWAY, NJ, US, vol. 26, no. 5, September 2007 (2007-09), - October 2007 (2007-10), pages 36-42, XP011192795, ISSN: 0739-5175, DOI: 10.1109/EMB.2007.901790

## Description

The present invention is directed to a medical data processing method in accordance with claim 1 for supporting determination of medical image data describing a spatial distribution of body tissue, a corresponding computer program, a computer running that program and a radiotherapy system or radiosurgery system comprising that computer.

Clinical Decision Support Systems which are image-based can be grouped in the following fashion:
- Treatment effect systems for implantation (bringing objects, substances or radiation into the body) or excision (eliminating existing objects such as tumours). While effects of implantation systems are not necessarily always image-verified (except for the deviation of planned trajectory/target and actually reached trajectory/target precision or changes in tissue composition/volume resulting from a radiation treatment) but have health effects mainly observable in patient metainformation (e.g. symptom reduction, increase in quality of life), the outcome of excision treatments can be directly measured on an image basis since post-intervention images in general show the volume reduction of the target object (e.g. tumour shrinking) or volume replacement (replacing tumour volume with a resection cavity/replacement tissue).
- Clinical decision support systems offer decision support for the clinician through inferences that are drawn from the presented case data (e.g. medical records) and an amount of input data that serves as the decision basis (e.g. loaded medical literature). In the case of structured data (e.g. database entries) or unstructured data (e.g. medical reports in text form) both are analyzed and weighted to come to a decision or a ranked list of suggestions, subsequently this inference is presented to the clinician. Medical decisions are not taken, though, because a "black box" says it would be a good idea, the reasoning behind the taken inference needs to be transparent. While for the sketched systems such views to illustrate the underlying reasoning already exist (e.g. listing all relevant medical literature reference in support of a said decision), outcome atlases that locate and relate statistical treatment outcome indicators in a patient population with a certain effect location in the body (e.g. where in the treatment something was implanted or some tissue excised) as well as the anatomical location and extent of the treated abnormality (e.g. tumour) do not yet have one accepted processing/viewing format to bring transparency to inferences drawn from such mass analysis of imaging data. The proposed invention aims to solve that problem by processing the data in a form that can be visualized at the point of inference giving / decision support to aid the clinician in accepting or declining the proposed decision support.

The following publications contain teachings from the state of the art:
WO2012054612 A1
WO2013043132 A1
WO2013117658 A1
WO2004096018 A2
WO2013123085 A1
WO2013130234 A1
P. C. De Witt Hamer, J. Hendriks, E. Mandonnet, F. Barkhof, A. H. Zwinderman, H. Duffau, Resection Probability Maps for Quality Assessment of Glioma Surgery without Brain Location Bias, PLOS One, vol. 8 (2013), issue no. 9, Sep 06 2013
WO 2011/068475 A1 discloses to input parameter data for a patient and, if the parameter data is incomplete, to predict the missing parameters for example on the basis of a predicted stroke atlas comprising parameter information which is comparable to the parameter data input for the patient.
EP 2 575 067 A2 discloses to reply on similarity threshold for comparing a current patient to a reference patient.
US 2010/125462 A1 teaches computation of a close cohort by determining a potential set of patients. This is done everytime a close cohort is to be computed.

Advantages, advantageous features, advantageous embodiments and advantageous aspects of the present invention are disclosed in the following. Different advantageous features can be combined in accordance with the invention wherever technically expedient and feasible. Specifically, a feature of one embodiment which has the same or a similar function to another feature of another embodiment can be exchanged with said other feature, and a feature of one embodiment which adds an additional function to another embodiment can for example be added to said other embodiment.

The present invention has for example application in image-guided surgery workflows where suggestions on a certain treatment course are to be weighted in the context of the present knowledge describing the success rate of such treatments in the past. Further applications can be considered for utilization in stereotaxy planning software (e.g. Trajectory Element or iPlan Stereotaxy) by Brainlab AG where optimal implantation treatments are planned on the basis of information from past successful implantations, radiation treatment planning software (e.g. Multi-Mets Element) and software designed to optimize the planning of vascular interventions (AngioBrush Element) such as AVMs (arterio-venous malformations).

### Exemplary Short Description of the Present Invention

In the following, a short description of the specific features of the present invention is given which shall not be understood to limit the invention only to the features or a combination of the features described in this section.

The present invention relates in one example aspect to a method for determining a suitable sample of sample patients for generating a patient-specific outcome atlas which describes the probability of success of treating a pathologic state by resection of tumour tissue or implanting a structure into a patient's body. The sample patients are determined by searching a database for an outcome atlas which is suitable in consideration of the patient's pathologic state, and the sample patients are selected from those which form a basis of the outcome atlas. The selection is carried out by comparing properties of the sample patients to corresponding properties of the patient. Sample patients which are associated with properties fulfilling a predetermined similarity criterion with regard to those properties of the patient are then selected as members of an adapted population, on the basis of which a patient-specific outcome atlas can - in one aspect of the invention - be generated. As part of the invention, a user can adjust the size of the sample population and thereby the degree of similarity (i.e. the predetermined similarity criterion) required for the sample patients to be selected as members of the adapted population.

### General Description of the Present Invention

In this section, a description of the general, in some cases particular preferred, features of the present invention is given.

In a first aspect, the invention relates to a method which is a data processing method, such as for example a medical data processing method, suitable for supporting determination of medical image data describing a spatial distribution of body tissue which is the subject of a medical procedure. The method for example comprises the following steps which are for instance constituted to be executed by a computer (further particularly, all of the steps of the method in accordance with the invention are constituted to be executed by a computer).

In a first embodiment, the disclosed method comprises acquiring outcome atlas data. The outcome atlas data describes (for example, defines) a general probability that a specific anatomical structure can (successfully) be the subject of the medical procedure. Being the subject of the medical procedure means for example being treated by means (e.g. by application) of the medical procedure. The treatment comprises for example an alteration (for example, a physical alteration) of the anatomical structure, such as at least one of for example removing (i.e. resecting) body tissue from the anatomical structure or from a body region adjacent (for example, directly neighbouring) the anatomical region, and supplementing the anatomical structure by e.g. conducting an implantation, such as implanting an implant into the anatomical structure or a body region adjacent (for example, directly neighbouring) the anatomical region. The general probability has been determined based on for example a statistical analysis of medical image data generated from a general population of human bodies. Alternatively or additionally, the medical image data can be generated based on (i.e. from) treatment effect simulations conducted on said medical image data. The general population for example suffers from the same pathological state (e.g. disease or injury) which is for example the same as the pathological state from which the patient is suffering. This allows deriving a statement for the probability of a specific outcome (e.g. quality of life, probability of complete healing, occurrence of collateral damage due to application of the medical procedure etc.) for the patient which is associated with (for example due to) carrying out the medical procedure on the patient (for example on the anatomical structure).

The general population is described (for example defined) for example by general population data describing at least one statistical feature of each member of the general population. In one exemplary embodiment, the general population data is also acquired by the disclosed method. The general population data serves as metadata for the outcome atlas data and describes for example at least one of the number of members of the general population, their respective pathological state, the way in which they were treated by application of a medical procedure, their age, gender, ethnicity, genetic information, body mass and body size.

In the first embodiment, the disclosed method comprises acquiring patient definition data describing at least one statistical feature of a patient who shall become the subject of the medical procedure. The statistical feature is for example at least one of the patient's pathological state, the patient's age, gender, ethnicity, body mass and for example body size or for example genetic information or for example the way in which that state was treated by application of a medical procedure.

In the first embodiment, the method comprises determining, based on the outcome atlas data and the general population data and the patient definition data, adapted population data. The adapted population data describes (for example defines) an adapted population of human bodies taken from the general population. The adapted population is for example a selection from the general population, i.e. the adapted population consists (only) of members of the general population, wherein it may comprise all of the members of the general population or less than the total of members of the general population. The adapted population is to be used (for example in future and not necessarily within the execution of the disclosed method) as a basis for generating adapted outcome atlas data. The adapted outcome atlas data describes (for example defines) an adapted probability
that the specific anatomical structure can be treated successfully by means of the medical procedure, which adapted probability is determined based on the adapted population.

The at least one statistical feature of the patient can be compared to the at least one statistical feature of each member of the general population, for example the statistical features are from the same category, i.e. describe comparable information. The adapted population data is determined by comparing the value of the at least one statistical feature of the patient to the value of the at least one statistical feature of each member of the general population. The method comprises determining, on the basis of the result of the comparison of the values of the at least one statistical feature, members of the general population which have a predetermined similarity to the patient as the members of the adapted population. As a further example, the adapted population data is determined based on user input of for example a predetermined degree of similarity between the members of the general population and the patient. The user input is for instance effected by operating a graphical user input such as a representation of a slider which is displayed for example in a statistical diagram illustrating the relationship between the similarity between the general population and the patient on the one hand, and the number of similar members of the general population on the other hand.

For example, the anatomical structure comprises tumour tissue and the general probability and the adapted probability each describe a probability that the tumour tissue can be successfully treated, for instance resected.

In a second exemplary embodiment, the disclosed method comprises for example at least one of the following features:
determining, based on the outcome atlas data and the adapted population data, adapted outcome data describing (for example, defining) a probability with which the specific anatomical structure can be successfully altered within a body of the patient;
acquiring patient medical image data describing (for example, defining) a medical image of the anatomical structure in the body of the patient;
determining, based on the adapted outcome data and the patient medical image data, alteration probability data describing (for example, defining) a probability of successfully altering the anatomical structure;
determining, based on the alteration probability data and the patient medical image data, alteration probability display data for displaying graphical information describing (for example, defining) the probability of successfully altering the anatomical structure simultaneously (i.e. at the same point in time on the same display apparatus or different display apparatus) with the patient medical image data.

In an optional variation of the second exemplary embodiment, the adapted outcome data is for example generated on the basis of medical image data so that the adapted outcome data can be displayed as a statistical map simultaneously with the medical image data.

In a third exemplary embodiment, the adapted population data is analysed to infer a contrast-to-noise-property which describes the minimum size of a subset of the adapted population data that carries statistical significance with respect to an available property, for example the outcome of carrying out the medical procedure on the patient.

In a fourth exemplary embodiment, the adapted population data is for example analysed to infer a contrast-to-noise-property which describes the minimum size (i.e. number of elements) of a subset of the adapted population data that carries statistical significance with respect to an available property, for example with respect to the outcome of carrying out the medical procedure on the patient.

In a fifth exemplary embodiment, the disclosed method comprises for example at least one of the following features:
acquiring patient medical image data describing a medical image of the anatomical structure in the body of the patient;
determining the adapted population data is determined further based on the patient medical image data.

In a first optional variation of the fifth embodiment, the outcome atlas data comprises the medical image data from which it has been generated, and wherein determining the adapted population data comprises comparing the patient medical image data to the outcome atlas data, for example selecting the adapted population such that the members of the adapted population are associated with medical image data having a predetermined degree of similarity to the patient medical image data. For example, comparing the patient medical image data to the outcome atlas data comprises running an image fusion algorithm on the patient medical image data and the outcome atlas data.

In a second optional variation of the fifth embodiment, the disclosed method comprises at least one of the following features:
determining patient feature vector data comprising the patient definition data and the result of an analysis of the patient medical image data with regard to morphological traits of the patient's anatomy;
determining the adapted population data further based on the patient feature vector data.

The term of vector data denotes for example a vector-type data structure in which the associated information is present and/or stored.

In a second aspect, the invention is also directed to a program which, when running on a computer, causes the computer to perform one or more or all of the method steps described herein and/or to a program storage medium on which the program is stored (for example in a non-transitory form) and/or to a computer comprising said program storage medium and/or to a (physical, for example electrical, for example technically generated) signal wave, for example a digital signal wave, carrying information which represents the program, for example the aforementioned program, which for example comprises code means which are adapted to perform any or all of the method steps described herein.

It is within the scope of the present invention to combine one or more features of one or more embodiments or aspects of the invention in order to form a new embodiment wherever this is technically expedient and/or feasible. Specifically, a feature of one embodiment which has the same or a similar function to another feature of another embodiment can be exchanged with said other feature, and a feature of one embodiment which adds an additional function to another embodiment can for example be added to said other embodiment.

The present invention in one example does not involve or for example comprise or encompass an invasive step which would represent a substantial physical interference with the body requiring professional medical expertise to be carried out and entailing a substantial health risk even when carried out with the required professional care and expertise. For example, the invention does not comprise a step of positioning a medical implant in order to fasten it to an anatomical structure or a step of fastening the medical implant to the anatomical structure or a step of preparing the anatomical structure for being fastened to the medical implant. More particularly, the invention does not involve or for example comprise or encompass any surgical or therapeutic activity. No surgical or therapeutic step is necessitated or implied by carrying out the invention.

### Definitions

In this section, definitions for specific terminology used in this disclosure are offered which also form part of the present disclosure.

Within the framework of the invention, computer program elements can be embodied by hardware and/or software (this includes firmware, resident software, micro-code, etc.). Within the framework of the invention, computer program elements can take the form of a computer program product which can be embodied by a computer-usable, for example computer-readable data storage medium comprising computer-usable, for example computer-readable program instructions, "code" or a "computer program" embodied in said data storage medium for use on or in connection with the instruction-executing system. Such a system can be a computer; a computer can be a data processing device comprising means for executing the computer program elements and/or the program in accordance with the invention, for example a data processing device comprising a digital processor (central processing unit or CPU) which executes the computer program elements, and optionally a volatile memory (for example a random access memory or RAM) for storing data used for and/or produced by executing the computer program elements. Within the framework of the present invention, a computer-usable, for example computer-readable data storage medium can be any data storage medium which can include, store, communicate, propagate or transport the program for use on or in connection with the instruction-executing system, apparatus or device. The computer-usable, for example computer-readable data storage medium can for example be, but is not limited to, an electronic, magnetic, optical, electromagnetic, infrared or semiconductor system, apparatus or device or a medium of propagation such as for example the Internet. The computer-usable or computer-readable data storage medium could even for example be paper or another suitable medium onto which the program is printed, since the program could be electronically captured, for example by optically scanning the paper or other suitable medium, and then compiled, interpreted or otherwise processed in a suitable manner. The data storage medium is for example a non-volatile data storage medium. The computer program product and any software and/or hardware described here form the various means for performing the functions of the invention in the example embodiments. The computer and/or data processing device can for example include a guidance information device which includes means for outputting guidance information. The guidance information can be outputted, for example to a user, visually by a visual indicating means (for example, a monitor and/or a lamp) and/or acoustically by an acoustic indicating means (for example, a loudspeaker and/or a digital speech output device) and/or tactilely by a tactile indicating means (for example, a vibrating element or a vibration element incorporated into an instrument). For the purpose of this document, a computer is a technical computer which for example comprises technical, for example tangible components, for example mechanical and/or electronic components. Any device mentioned as such in this document is a technical and for example tangible device.

The method in accordance with the invention is for example a data processing method. The data processing method is for example performed using technical means, for example a computer. The data processing method is for example constituted to be executed by or on a computer and for example is executed by or on the computer. For example, all the steps or merely some of the steps (i.e. less than the total number of steps) of the method in accordance with the invention can be executed by a computer. The computer for example comprises a processor and a memory in order to process the data, for example electronically and/or optically. The calculating steps described are for example performed by a computer. Determining steps or calculating steps are for example steps of determining data within the framework of the technical data processing method, for example within the framework of a program. A computer is for example any kind of data processing device, for example electronic data processing device. A computer can be a device which is generally thought of as such, for example desktop PCs, notebooks, netbooks, etc., but can also be any programmable apparatus, such as for example a mobile phone or an embedded processor. A computer can for example comprise a system (network) of "sub-computers", wherein each sub-computer represents a computer in its own right. The term "computer" includes a cloud computer, for example a cloud server. The term "cloud computer" includes a cloud computer system which for example comprises a system of at least one cloud computer and for example a plurality of operatively interconnected cloud computers such as a server farm. Such a cloud computer is for example connected to a wide area network such as the world wide web (WWW) and located in a so-called cloud of computers which are all connected to the world wide web. Such an infrastructure is used for "cloud computing", which describes computation, software, data access and storage services which do not require the end user to know the physical location and/or configuration of the computer delivering a specific service. For example, the term "cloud" is used in this respect as a metaphor for the Internet (world wide web). For example, the cloud provides computing infrastructure as a service (laaS). The cloud computer can function as a virtual host for an operating system and/or data processing application which is used to execute the method of the invention. The cloud computer is for example an elastic compute cloud (EC2) as provided by Amazon Web Services™. A computer for example comprises interfaces in order to receive or output data and/or perform an analogue-to-digital conversion. The data are for example data which represent physical properties and/or which are generated from technical signals. The technical signals are for example generated by means of (technical) detection devices (such as for example devices for detecting marker devices) and/or (technical) analytical devices (such as for example devices for performing imaging methods), wherein the technical signals are for example electrical or optical signals. The technical signals for example represent the data received or outputted by the computer. The computer is for example operatively coupled to a display device which allows information outputted by the computer to be displayed, for example to a user. One example of a display device is an augmented reality device (also referred to as augmented reality glasses) which can be used as "goggles" for navigating. A specific example of such augmented reality glasses is Google Glass (a trademark of Google, Inc.). An augmented reality device can be used both to input information into the computer by user interaction and to display information outputted by the computer. Another example of a display device would be a standard computer monitor comprising for example a liquid crystal display operatively coupled to the computer for receiving display control data from the computer for generating signals used to display image information content on the display device. A specific embodiment of such a computer monitor is a digital lightbox. The monitor may also be the monitor of a portable, for example handheld, device such as a smart phone or personal digital assistant or digital media player.

The expression "acquiring data" for example encompasses (within the framework of a data processing method) the scenario in which the data are determined by the data processing method or program. Determining data for example encompasses measuring physical quantities and transforming the measured values into data, for example digital data, and/or computing the data by means of a computer and for example within the framework of the method in accordance with the invention. The meaning of "acquiring data" also for example encompasses the scenario in which the data are received or retrieved by the data processing method or program, for example from another program, a previous method step or a data storage medium, for example for further processing by the data processing method or program. The expression "acquiring data" can therefore also for example mean waiting to receive data and/or receiving the data. The received data can for example be inputted via an interface. The expression "acquiring data" can also mean that the data processing method or program performs steps in order to (actively) receive or retrieve the data from a data source, for instance a data storage medium (such as for example a ROM, RAM, database, hard drive, etc.), or via the interface (for instance, from another computer or a network). The data can be made "ready for use" by performing an additional step before the acquiring step. In accordance with this additional step, the data are generated in order to be acquired. The data are for example detected or captured (for example by an analytical device). Alternatively or additionally, the data are inputted in accordance with the additional step, for instance via interfaces. The data generated can for example be inputted (for instance into the computer). In accordance with the additional step (which precedes the acquiring step), the data can also be provided by performing the additional step of storing the data in a data storage medium (such as for example a ROM, RAM, CD and/or hard drive), such that they are ready for use within the framework of the method or program in accordance with the invention. The step of "acquiring data" can therefore also involve commanding a device to obtain and/or provide the data to be acquired. For example, the acquiring step does not involve an invasive step which would represent a substantial physical interference with the body, requiring professional medical expertise to be carried out and entailing a substantial health risk even when carried out with the required professional care and expertise. For example, the step of acquiring data, for example determining data, does not involve a surgical step and for example does not involve a step of treating a human or animal body using surgery or therapy. In order to distinguish the different data used by the present method, the data are denoted (i.e. referred to) as "XY data" and the like and are defined in terms of the information which they describe, which is then for example referred to as "XY information" and the like.

In the field of medicine, imaging methods (also called imaging modalities and/or medical imaging modalities) are used to generate image data (for example, two-dimensional or three-dimensional image data) of anatomical structures (such as soft tissues, bones, organs, etc.) of the human body. The term "medical imaging methods" is understood to mean (advantageously apparatus-based) imaging methods (so-called medical imaging modalities and/or radiological imaging methods) such as for instance computed tomography (CT) and cone beam computed tomography (CBCT, for example volumetric CBCT), x-ray tomography, magnetic resonance tomography (MRT or MRI), conventional x-ray, sonography and/or ultrasound examinations, and positron emission tomography. The image data thus generated is also termed "medical imaging data". Analytical devices are for example used to generate the image data in apparatus-based imaging methods. The imaging methods are for example used for medical diagnostics, to analyse the anatomical body in order to generate images which are described by the image data. The imaging methods are also for example used to detect pathological changes in the human body.

Image fusion can be elastic image fusion or rigid image fusion. In the case of rigid image fusion, the relative position between the pixels of a 2D image and/or voxels of a 3D image is fixed, while in the case of elastic image fusion, the relative positions are allowed to change.

In this application, the term "image morphing" is also used as an alternative to the term "elastic image fusion", but with the same meaning.

Elastic fusion transformations (for example, elastic image fusion transformations) are for example designed to enable a seamless transition from one dataset (for example a first dataset such as for example a first image) to another dataset (for example a second dataset such as for example a second image). The transformation is for example designed such that one of the first and second datasets (images) is deformed, for example in such a way that corresponding structures (for example, corresponding image elements) are arranged at the same position as in the other of the first and second images. The deformed (transformed) image which is transformed from one of the first and second images is for example as similar as possible to the other of the first and second images. For example, (numerical) optimisation algorithms are applied in order to find the transformation which results in an optimum degree of similarity. The degree of similarity is for example measured by way of a measure of similarity (also referred to in the following as a "similarity measure"). The parameters of the optimisation algorithm are for example vectors of a deformation field. These vectors are determined by the optimisation algorithm in such a way as to result in an optimum degree of similarity. Thus, the optimum degree of similarity represents a condition, for example a constraint, for the optimisation algorithm. The bases of the vectors lie for example at voxel positions of one of the first and second images which is to be transformed, and the tips of the vectors lie at the corresponding voxel positions in the transformed image. A plurality of these vectors are preferably provided, for instance more than twenty or a hundred or a thousand or ten thousand, etc. For example, there are (other) constraints on the transformation (deformation), for example in order to avoid pathological deformations (for instance, all the voxels being shifted to the same position by the transformation). These constraints include for example the constraint that the transformation is regular, which for example means that a Jacobian determinant calculated from a matrix of the deformation field (for example, the vector field) is larger than zero, and also the constraint that the transformed (deformed) image is not self-intersecting and for example that the transformed (deformed) image does not comprise faults and/or ruptures. The constraints include for example the constraint that if a regular grid is transformed simultaneously with the image and in a corresponding manner, the grid is not allowed to interfold at any of its locations. The optimising problem is for example solved iteratively, for example by means of an optimisation algorithm which is for example a first-order optimisation algorithm, for example a gradient descent algorithm. Other examples of optimisation algorithms include optimisation algorithms which do not use derivations, such as the downhill simplex algorithm, or algorithms which use higher-order derivatives such as Newton-like algorithms. The optimisation algorithm preferably performs a local optimisation. If there are a plurality of local optima, global algorithms such as simulated annealing or generic algorithms can be used. In the case of linear optimisation problems, the simplex method can for instance be used.

In the steps of the optimisation algorithms, the voxels are for example shifted by a magnitude in a direction such that the degree of similarity is increased. This magnitude is preferably less than a predefined limit, for instance less than one tenth or one hundredth or one thousandth of the diameter of the image, and for example about equal to or less than the distance between neighbouring voxels. Large deformations can be implemented, for instance due to a high number of (iteration) steps.

The determined elastic fusion transformation can for example be used to determine a degree of similarity (or similarity measure, see above) between the first and second datasets (first and second images). To this end, the deviation between the elastic fusion transformation and an identity transformation is determined. The degree of deviation can for instance be calculated by determining the difference between the determinant of the elastic fusion transformation and the identity transformation. The higher the deviation, the lower the similarity, hence the degree of deviation can be used to determine a measure of similarity.

A measure of similarity can for example be determined on the basis of a determined correlation between the first and second datasets.

Preferably, atlas data is acquired which describes (for example defines, more particularly represents and/or is) a general three-dimensional shape of the anatomical body part. The atlas data therefore represents an atlas of the anatomical body part. An atlas typically consists of a plurality of generic models of objects, wherein the generic models of the objects together form a complex structure. For example, the atlas constitutes a statistical model of a patient's body (for example, a part of the body) which has been generated from anatomic information gathered from a plurality of human bodies, for example from medical image data containing images of such human bodies. In principle, the atlas data therefore represents the result of a statistical analysis of such medical image data for a plurality of human bodies. This result can be output as an image - the atlas data therefore contains or is comparable to medical image data. Such a comparison can be carried out for example by applying an image fusion algorithm which conducts an image fusion between the atlas data and the medical image data. The result of the comparison can be a measure of similarity between the atlas data and the medical image data.

The human bodies, the anatomy of which serves as an input for generating the atlas data, advantageously share a common feature such as at least one of gender, age, ethnicity, body measurements (e.g. size and/or mass) and pathologic state. The anatomic information describes for example the anatomy of the human bodies and is extracted for example from medical image information about the human bodies. The atlas of a femur, for example, can comprise the head, the neck, the body, the greater trochanter, the lesser trochanter and the lower extremity as objects which together make up the complete structure. The atlas of a brain, for example, can comprise the telencephalon, the cerebellum, the diencephalon, the pons, the mesencephalon and the medulla as the objects which together make up the complex structure. One application of such an atlas is in the segmentation of medical images, in which the atlas is matched to medical image data, and the image data are compared with the matched atlas in order to assign a point (a pixel or voxel) of the image data to an object of the matched atlas, thereby segmenting the image data into objects.

### Description of the Figures

In the following, the invention is described with reference to the enclosed figures which represent preferred embodiments of the invention. The scope of the invention is not however limited to the specific features disclosed in the figures, wherein
- Fig. 1: is a flow diagram showing i.a. the steps of acquiring a single patient image and patient meta-information; and
- Fig. 2: illustrates a sample selection slider on the basis of patient similarity with the sample and number of cases in the population database in total.

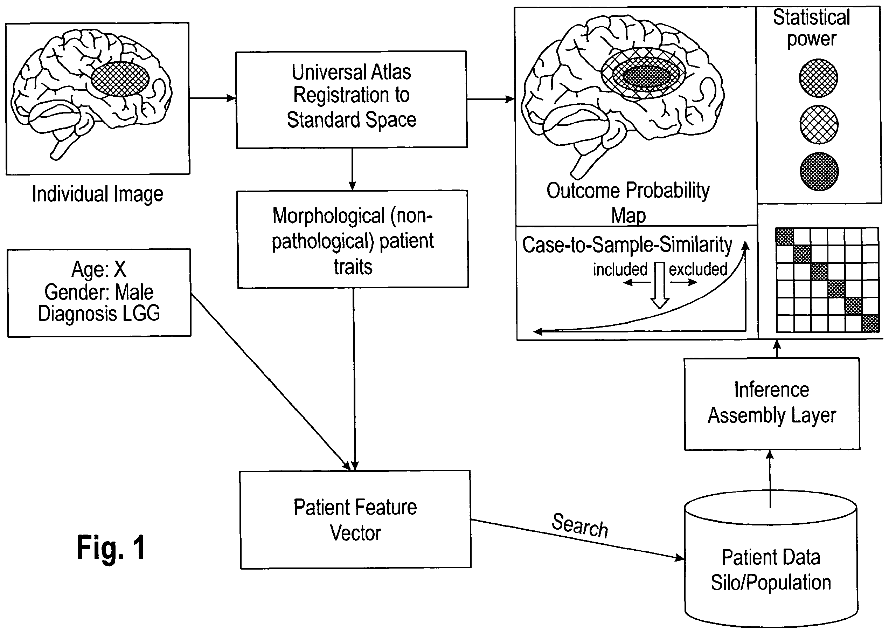

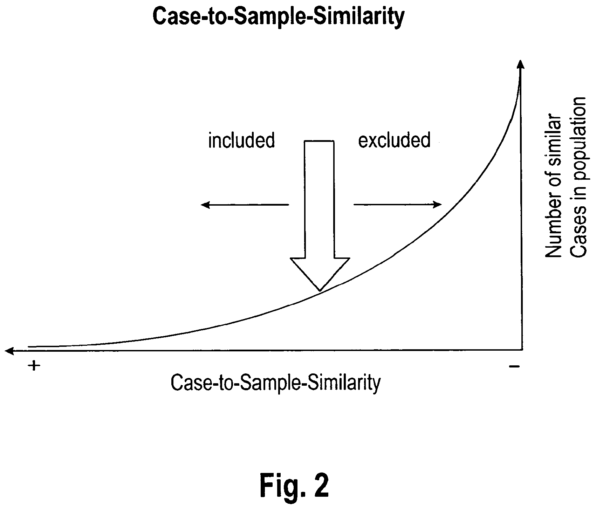

In a realization of the disclosed method, determination of patient traits from features originating in patient meta-information (e.g. age, gender etc., which are acquired as the patient definition data) is combined with patient traits determined from the imaging data (i.e. the outcome atlas data) in order to define a feature vector describing the medical case at hand. The patient population already loaded into the inference system (i.e. the acquired general population data) is compared with the patient at hand (as defined by the patient definition data) and the amount of similar cases (which constitutes at least a part of the adapted population data) is selected which is necessary for a statistically valid inference. Image-based and not image-based aspects of case-to-sample-similarity are displayed and by using a GUI element such as a slider (cf. the large arrow in Fig. 2 indicating the division between the cases from the general population included in and excluded from, respectively, the adapted population), the sample size (i.e. the size of the adapted population) can be increased at the cost of a reduction of case-to-sample-similarity. Statistical power of the drawn inference is displayed similarly but will grow with the sample size utilized. The fast calculation of case-adaptive inferences (i.e. outcome atlases) is supported by the outlined (see Fig. 1) architecture of utilizing intermediate inference repositories from which pre-processed results can be quickly assembled into adapted maps.

Fig. 1 illustrates that, for determining the patient feature vector, the individual image defined by medical image data describing the patient at hand is registered to an atlas to transfer it into a standard coordinate space and to determine certain morphological traits of the patient which are then stored in the patient feature vector. The outcome atlas data stored in the patient data silo is then searched for members of the general population which fulfil at least one predetermined criterion for a similarity with at least one specific characteristic (i.e. statistical feature) stored in the patient feature vector.

An outcome atlas such as a resectability atlas fulfils the purpose of highlighting the probability with which a tumour can be successfully resected. Calculating the difference between all available preoperative tumour volumes and all available residual postoperative tumour volumes which remain after a surgical intervention on the tumour, volumes can be calculated that represent the statistically significant average volume of residual tumour. These volumes are neither directly visible from the preoperative nor the postoperative images but represent an intermediary result. These are assembled into the final outcome atlas (embodying the outcome atlas data) by collecting all of these volumes in one common frame of reference and expressing the resectability as a local probability. This last inference assembly step can be tuned so as to only include patients from the available patient data population which correspond to the case at hand with respect to certain similarity features (e.g. age and gender, presence or absence of genetic properties or morphological image-properties such as e.g. grey/white-matter tissue ratio).

Statistical power for a selected subsample of all available patient data is calculated by analysing the effect size of the treatment (e.g. surgical resection) in response to the indication (e.g. low-grade glioma). In the case of resection the effect is large (tumour disappears), which might not be the case for all analysed treatments (e.g. radiation treatment or a certain pharmacological agent lead probably only to a slight shrinkage in some cases). For treatments with weaker of more distributed effects more cases need to be assembled for the final outcome map to be of statistical significance (e.g. if the shrinkage is only minor that effect has to be put in contrast with "natural" variations or measurement imprecisions that occur in the data and might obscure minimal but significant effects).

With this system the inference logic behind an image-based recommendation/clinical decision support becomes transparent at the point where the clinician receives the information - and can decide to accept or decline this information. The system permits the user to understand whether the data presently in the clinical decision support systems really relates to the case at hand or not - which in turn allows for an understanding of how "regular" the current case seems to be.

The image-based and non-image-based patient similarity selection/display engine which selects the input sample for the outcome atlas and the intermediate inference layer database from which the relevant results are selected for result and statistical validity calculation and final display. The inference assembly layer utilizes precalculated information from the intermediate inference layer database to assemble the relevant results for the case into an image (e.g. an outcome atlas). The statistical power calculator utilizes sample size and effect size on the basis of the target volume in relation to the sample-covered image volumes.

## Claims

1. A medical data processing method for supporting determination of medical image data describing a spatial distribution of body tissue which is the subject of a medical procedure, the method comprising the following steps which are constituted to be executed by a computer:
a) acquiring outcome atlas data describing a general probability that a specific anatomical structure can be treated successfully by means of the medical procedure, the general probability having been determined based on a statistical analysis of medical image data and/or treatment effect simulations conducted on said medical image data generated from a general population of human bodies;
b) acquiring general population data describing at least one statistical feature of each member of the general population, wherein the at least one statistical feature of a patient can be compared to the at least one statistical feature of each member of the general population;
c) acquiring patient definition data describing at least one statistical feature of a patient who shall become the subject of the medical procedure, wherein the at least one statistical feature of each member of the general population and the at least one statistical feature of the patient pertain to at least one of genetic information and information about the gender, age, ethnicity, mass and pathological state of the members of the general population and the patient, respectively;
d) determining, based on the outcome atlas data and the general population data and the patient definition data, adapted population data describing an adapted population of human bodies taken from the general population, wherein the adapted population data is determined by comparing the value of the at least one statistical feature of the patient to the value of the at least one statistical feature of each member of the general population and wherein members of the general population which have a predetermined similarity to the patient are determined as the members of the adapted population and on the basis of the result of the comparison of the values of the at least one statistical feature, wherein the adapted population is to be used as a basis for generating adapted outcome atlas data describing an adapted probability that the specific anatomical structure can be treated successfully by means of the medical procedure, which adapted probability is determined based on the adapted population.

2. The method according to the preceding claim, comprising:
determining, based on the outcome atlas data and the adapted population data, adapted outcome data describing a probability with which the specific anatomical structure can be successfully altered within a body of a patient;
acquiring patient medical image data describing a medical image of the anatomical structure in the body of the patient;
determining, based on the adapted outcome data and the patient medical image data, alteration probability data describing a probability of successfully altering the anatomical structure;
determining, based on the alteration probability data and the patient medical image data, alteration probability display data for displaying graphical information describing the probability of successfully altering the anatomical structure simultaneously with the patient medical image data.

3. The method according to the preceding claim, wherein the adapted outcome data is generated on the basis of medical image data so that the adapted outcome data can be displayed as a statistical map simultaneously with the medical image data.

4. The method according to any one of the preceding claims, wherein the adapted population data is analysed to infer a contrast-to-noise-property which describes the minimum size of a subset of the adapted population data that carries statistical significance with respect to an available property, for example the outcome of carrying out the medical procedure on the patient.

5. The method according to any one of the preceding claims, wherein the adapted population data is determined based on user input of for example a predetermined degree of similarity between the members of the general population and the patient.

6. The method according to the preceding claim, wherein the user input is effected by operating a graphical user input such as for example a representation of a slider for example in a statistical diagram illustrating the relationship between the similarity between the general population and the patient, and the number of similar members of the general population.

7. The method according to any one of the preceding claims, wherein the anatomical structure comprises tumour tissue and wherein the general probability and the adapted probability each describe a probability that the tumour tissue can be successfully resected.

8. The method according to any one of the preceding claims, comprising:
acquiring patient medical image data describing a medical image of the anatomical structure in the body of the patient,
wherein the adapted population data is determined further based on the patient medical image data.

9. The method according to the preceding claim, wherein the outcome atlas data comprises the medical image data from which it has been generated, and wherein determining the adapted population data comprises comparing the patient medical image data to the outcome atlas data, for example selecting the adapted population such that the members of the adapted population are associated with medical image data having a predetermined degree of similarity to the patient medical image data.

10. The method according to the preceding claim, wherein comparing the patient medical image data to the outcome atlas data comprises running an image fusion algorithm on the patient medical image data and the outcome atlas data.

11. The method according to any one of the three immediately preceding claims, comprising:
determining patient feature vector data comprising the patient definition data and the result of an analysis of the patient medical image data with regard to morphological traits of the patient's anatomy;
determining the adapted population data further based on the patient feature vector data.

12. A program which, when running on a computer or when loaded onto a computer, causes the computer to perform the method steps according to any one of the preceding claims.

13. A program storage medium on which the program according to the preceding claim is stored.

14. A computer on which the program according to claim 12 is running or into the memory of which the program according to claim 12 is loaded.

15. A signal wave carrying information which represents the program according to claim 12, which comprises code means which are adapted to perform the method steps according to any one of claims 1 to 11.

## Patentansprüche

1. Medizinisches Datenverarbeitungsverfahren zum Unterstützen der Ermittlung medizinischer Bilddaten, die eine räumliche Verteilung von Körpergewebe beschreiben, das Gegenstand einer medizinischen Prozedur ist, wobei das Verfahren die folgenden Schritte enthält, die eingerichtet sind, von einem Computer ausgeführt zu werden:
a) Einlesen von Ergebnisatlasdaten, die eine allgemeine Wahrscheinlichkeit dafür, dass eine spezifische anatomische Struktur erfolgreich mittels der medizinischen Prozedur behandelt werden kann, eingelesen, wobei die allgemeine Wahrscheinlichkeit basierend auf einer statistischen Analyse medizinischer Bilddaten und/oder Behandlungseffektsimulationen, die auf den medizinischen Bilddaten, die aus einer allgemeinen Population menschlicher Körper erzeugt worden sind, durchgeführt worden sind, ermittelt worden ist;
b) Einlesen von Allgemeinpopulationsdate, die wenigstens ein statistisches Merkmal jedes Mitglieds der allgemeinen Population beschreiben, wobei das wenigstens eine statistische Merkmal eines Patienten mit wenigstens einem statistischen Merkmal jedes Mitglieds der allgemeinen Population verglichen werden kann;
c) Einlesen von Patientendefinitionsdate, die wenigstens ein statistisches Merkmal eines Patienten, der Gegenstand der medizinischen Prozedur werden soll, beschreiben, wobei das wenigstens eine statistische Merkmal jedes Mitglieds der allgemeinen Population und das wenigstens statistische Merkmal des Patienten sich auf wenigstens aus genetischen Informationen und Informationen über das Geschlecht, Alter, die Ethnizität, die Masse und den pathologischen Zustand des Mitglieds der allgemeinen Population bzw. des Patienten beziehen;
d) Ermitteln, basierend auf den Ergebnisatlasdaten und den allgemeinen Populationsdaten und den Patientendefinitionsdaten, angepasster Populationsdaten, die eine angepasste Population menschlicher Körper, die der allgemeinen Population entnommen sind, beschreiben, wobei die angepassten Populationsdaten durch Vergleichen des Werts des wenigstens einen statistischen Merkmals des Patienten mit dem Wert des wenigstens einen statistischen Merkmals jedes Mitglieds der allgemeinen Population ermittelt werden, und wobei Mitglieder der allgemeinen Population, die eine vorbestimmte Ähnlichkeit zu dem Patienten aufweisen, als Mitglieder der angepassten Population und auf Grundlage des Ergebnisses des Vergleichs des Werts des wenigstens einen statistischen Merkmals bestimmt werden, wobei die angepasste Population als Grundlage für die Erzeugung angepasster Ergebnisatlasdaten verwendet werden soll, die eine angepasste Wahrscheinlichkeit dafür beschreiben, dass die spezifische anatomische Struktur erfolgreich mittels der medizinischen Prozedur behandelt werden kann, wobei die angepasste Wahrscheinlichkeit basierend auf der angepassten Population ermittelt wird.

2. Verfahren gemäß dem vorhergehenden Anspruch, enthaltend:
Ermitteln, basierend auf den Ergebnisatlasdaten und den angepassten Populationsdaten, angepasste Ergebnisdaten, die eine Wahrscheinlichkeit, mit der die spezifische anatomische Struktur erfolgreich innerhalb eines Körpers eines Patienten verändert werden kann, beschreiben;
Einlesen medizinischer Patientenbilddaten, die ein medizinisches Bild einer anatomischen Struktur in dem Körper des Patienten beschreiben;
Ermitteln, basierend auf den angepassten Ergebnisdaten und den medizinischen Patientendaten, von Veränderungswahrscheinlichkeitsdaten, die eine Wahrscheinlichkeit für ein erfolgreiches Verändern der anatomischen Struktur beschreiben;
Ermitteln, basierend auf den Veränderungswahrscheinlichkeitsdaten und den medizinischen Patientenbilddaten, von Veränderungswahrscheinlichkeitsanzeigedaten zum mit den medizinischen Patientenbilddaten gleichzeitigen Anzeigen graphischer Informationen, die die Wahrscheinlichkeit für ein erfolgreiches Verändern der anatomischen Struktur beschreiben.

3. Verfahren gemäß dem vorhergehenden Anspruch, bei dem die angepassten Ergebnisdaten auf Grundlage der medizinischen Bilddaten ermittelt werden, so dass die angepassten Ergebnisdaten als statistische Übersichtskarte gleichzeitig mit den medizinischen Bilddaten angezeigt werden können.

4. Verfahren gemäß irgendeinem der vorhergehenden Ansprüche, bei dem die angepassten Populationsdaten so analysiert werden, dass eine Kontrast-zu-Rauschen-Eigenschaft abgeleitet werden kann, die die minimale Größe einer Untermenge der angepassten Populationsdaten beschreiben, die eine statistische Signifikanz in Bezug auf eine vorhandene Eigenschaft in sich tragen, zum Beispiel in Bezug auf das Ergebnis des Ausführens der medizinischen Prozedur an dem Patienten.

5. Verfahren gemäß irgendeinem der vorhergehenden Ansprüche, bei dem die angepassten Populationsdaten basierend auf einer Benutzereingabe zum Beispiel eines vorbestimmten Grads an Ähnlichkeit zwischen den Mitgliedern allgemeiner Population und dem Patienten ermittelt werden.

6. Verfahren gemäß dem vorhergehenden Anspruch, bei dem die Benutzereingabe durch Bedienen einer graphischen Benutzereingabe wie zum Beispiel einer Wiedergabe eines Schiebers in zum Beispiel einem statistischen Diagramm, das die Beziehung zwischen der Ähnlichkeit zwischen der allgemeinen Population und dem Patienten und der Anzahl ähnlicher Mitglieder der allgemeinen Population darstellt, bewirkt wird.

7. Verfahren gemäß irgendeinem der vorhergehenden Ansprüche, bei dem die anatomische Struktur Tumorgewebe enthält und bei dem die allgemeine Wahrscheinlichkeit und die angepasste Wahrscheinlichkeit jeweils eine Wahrscheinlichkeit dafür beschreiben, dass das Tumorgewebe erfolgreich reseziert werden kann.

8. Verfahren gemäß irgendeinem der vorhergehenden Ansprüche, enthaltend:
Einlesen von medizinischen Patientenbilddaten, die ein medizinisches Bild der anatomischen Struktur in dem Körper des Patienten beschreiben,
wobei die angepassten Populationsdaten ferner auf Grundlage der medizinischen Patientenbilddaten ermittelt werden.

9. Verfahren gemäß dem vorhergehenden Anspruch, bei dem die Ergebnisatlasdaten die medizinischen Bilddaten enthalten, aus denen sie erzeugt worden sind, und bei dem das Ermitteln der angepassten Populationsdaten ein Vergleichen der medizinischen Patientenbilddaten mit den Ergebnisatlasdaten, zum Beispiel ein Auswählen der angepassten Population in der Art, dass die Mitglieder der angepassten Population den medizinischen Bilddaten zugeordnet sind, die einen vorbestimmten Grad an Ähnlichkeit mit den medizinischen Patientenbilddaten aufweisen, enthält.

10. Verfahren gemäß dem vorhergehenden Anspruch, bei dem das Vergleichen der medizinischen Patientenbilddaten mit den Ergebnisatlasdaten umfasst, dass ein Bildfusionsalgorithmus auf den medizinischen Patientenbilddaten und den Ergebnisatlasdaten ausgeführt wird.

11. Verfahren gemäß irgendeinem der drei unmittelbar vorgehenden Ansprüche, enthaltend:
Ermitteln von Patientenmerkmalsvektordaten, die die Patientendefinitionsdaten und das Ergebnis einer Analyse der medizinischen Patientenbilddaten in Bezug auf morphologische Eigenschaften der Anatomie des Patienten enthalten;
Ermitteln der angepassten Populationsdaten ferner auf Grundlage der Patientenmerkmalsvektordaten.

12. Programm, das, wenn es auf einem Computer ausgeführt wird oder auf einen Computer geladen wird, den Computer dazu veranlasst, die Verfahrensschritte gemäß irgendeinem der vorhergehenden Ansprüche auszuführen.

13. Programmspeichermedium, auf dem das Programm gemäß dem vorhergehenden Anspruch gespeichert ist.

14. Computer, auf dem das Programm gemäß Anspruch 12 läuft oder in dessen Speicher das Programm gemäß Anspruch 12 geladen ist.

15. Signalwelle, die Informationen trägt, die das Programm gemäß Anspruch 12 repräsentieren, die Codemittel enthält, die angepasst sind, die Verfahrensschritte gemäß irgendeinem der Ansprüche 1 bis 11 auszuführen.

## Revendications

1. Procédé de traitement de données médicales destiné à soutenir la détermination de données d'image médicale décrivant une répartition spatiale de tissu corporel qui fait l'objet d'une procédure médicale, le procédé comprenant les étapes suivantes qui sont conçues pour être exécutées par un ordinateur :
a) l'acquisition de données d'atlas de sortie décrivant une probabilité générale qu'une structure anatomique spécifique peut être traitée avec succès au moyen de la procédure médicale, la probabilité générale ayant été déterminée en fonction d'une analyse statistique de données d'image médicale et/ou de simulations d'effet de traitement effectuées sur lesdites données d'image médicale générées à partir d'une population générale de corps humains ;
b) l'acquisition de données de population générale décrivant au moins une caractéristique statistique de chaque élément de la population générale, l'au moins une caractéristique statistique d'un patient pouvant être comparée à l'au moins une caractéristique statistique de chaque élément de la population générale ;
c) l'acquisition de données de définition de patient décrivant au moins une caractéristique statistique d'un patient qui devra faire l'objet de la procédure médicale, l'au moins une caractéristique statistique de chaque élément de la population générale et l'au moins une caractéristique statistique du patient portent sur des informations génétiques et/ou des informations sur le genre, l'âge, l'appartenance ethnique, la masse et l'état pathologique des éléments de la population générale et du patient, respectivement ;
d) la détermination, en fonction des données d'atlas de sortie et des données de population générale et des données de définition de patient, de données de population adaptée décrivant une population adaptée de corps humains provenant de la population générale, les données de population adaptée étant déterminées par la comparaison de la valeur de l'au moins une caractéristique statistique du patient à la valeur de l'au moins une caractéristique statistique de chaque élément de la population générale et des éléments de la population générale qui présentent une similitude prédéterminée au patient étant déterminés comme éléments de la population adaptée et en fonction du résultat de la comparaison des valeurs de l'au moins une caractéristique statistique, la population adaptée devant servir de base pour la génération de données d'atlas de sortie adaptée décrivant une probabilité adaptée que la structure anatomique peut être traitée avec succès au moyen de la procédure médicale, laquelle probabilité adaptée est déterminée en fonction de la population adaptée.

2. Procédé selon la revendication précédente, comprenant :
la détermination, en fonction des données d'atlas de sortie et des données de population adaptée, de données de sortie adaptée décrivant une probabilité selon laquelle la structure anatomique spécifique peut être modifiée avec succès dans le corps d'un patient ;
l'acquisition de données d'image médicale de patient décrivant une image médicale de la structure anatomique dans le corps du patient ;
la détermination, en fonction des données de sortie adaptée et des données d'image médicale de patient, de données de probabilité de modification décrivant une probabilité de modification avec succès de la structure anatomique ;
la détermination, en fonction des données de probabilité de modification et des données d'image médicale de patient, de données d'affichage de probabilité de modification permettant d'afficher des informations graphiques décrivant la probabilité de modifier avec succès la structure anatomique de manière simultanée avec les données d'image médicale de patient.

3. Procédé selon la revendication précédente, dans lequel les données de sortie adaptée sont générées en fonction de données d'image médicale de manière que les données de sortie adaptée peuvent être affichées comme carte statistique de manière simultanée avec les données d'image médicale.

4. Procédé selon l'une quelconque des revendications précédentes, dans lequel les données de population adaptée sont analysées pour déduire une propriété de contraste au bruit qui décrit la dimension minimale d'un sous-ensemble des données de population adaptée qui revêt une signification statistique par rapport à une propriété disponible, par exemple le résultat de la réalisation de la procédure médicale sur le patient.

5. Procédé selon l'une quelconque des revendications précédentes, dans lequel les données de population adaptée sont déterminées en fonction d'une entrée utilisateur de par exemple un degré prédéterminé de similitude entre les éléments de la population générale et le patient.

6. Procédé selon la revendication précédente, dans lequel l'entrée utilisateur est effectuée par la réalisation d'une entrée utilisateur graphique comme par exemple une représentation d'un curseur par exemple dans un schéma statistique illustrant la relation entre la similitude entre la population générale et le patient, et le nombre d'éléments similaires de la population générale.

7. Procédé selon l'une quelconque des revendications précédentes, dans lequel la structure anatomique comprend un tissu tumoral et dans lequel la probabilité générale et la probabilité adaptée décrivent chacune une probabilité que le tissu tumoral peut être réséqué avec succès.

8. Procédé selon l'une quelconque des revendications précédentes, comprenant :
l'acquisition de données d'image médicale de patient décrivant une image médicale de la structure anatomique dans le corps du patient,
dans lequel les données de population adaptée sont déterminées en outre en fonction des données d'image médicale de patient.

9. Procédé selon la revendication précédente, dans lequel les données d'atlas de sortie comprennent les données d'image médicale à partir desquelles elles ont été générées, et dans lequel la détermination des données de population adaptée comprend la comparaison des données d'image médicale de patient aux données d'atlas de sortie, par exemple la sélection de la population adaptée de manière que les éléments de la population adaptée sont associés aux données d'image médicale présentant un degré prédéterminé de similitude aux données d'image médicale de patient.

10. Procédé selon la revendication précédente, dans lequel la comparaison des données d'image médicale de patient aux données d'atlas de sortie comprend l'exécution d'un algorithme de fusion d'images sur les données d'image médicale de patient et les données d'atlas de sortie.

11. Procédé selon l'une quelconque des trois revendications immédiatement précédentes, comprenant :
la détermination de données vectorielles de caractéristique de patient comprenant les données de définition de patient et le résultat d'une analyse des données d'image médicale de patient par rapport aux caractères morphologiques de l'anatomie du patient ;
la détermination des données de population adaptée en outre fonction des données
vectorielles de caractéristique de patient.

12. Programme qui, lorsqu'exécuté sur un ordinateur ou lorsque chargé sur un ordinateur, amène l'ordinateur à effectuer les étapes de procédé selon l'une quelconque des revendications précédentes.

13. Support de stockage de programme sur lequel le programme selon la revendication précédente est stocké.

14. Ordinateur sur lequel le programme selon la revendication 12 est exécuté ou dans la mémoire duquel le programme selon la revendication 12 est chargé.

15. Onde de signal transportant des informations qui représentent le programme selon la revendication 12, qui comprend des moyens de code qui sont conçus pour effectuer les étapes de procédé selon l'une quelconque des revendications 1 à 11.
